# EUROPEAN PATENT APPLICATION

(11) **EP 4 635 558 A2**
(43) Date of publication of application: **22.10.2025**
(21) Application number: 25200469.2
(22) Date of filing: 10.11.2022
(51) Int. Cl.: A61N 1/375

(54) **IMPLANTABLE MEDICAL DEVICE COMPRISING AN ELECTRODE DEVICE HAVING A STEROID RESERVOIR**

(30) Priority: 18.11.2021 EP 21208929
(62) Divisional of application: 22817568.3
(71) Applicant: BIOTRONIK SE & Co. KG, 12359 Berlin (DE)
(72) Inventor: KOLBERG, Gernot, 12161 Berlin (DE); DOHMEN, Daniel, San Francisco, 94109 (US); HILLEBRAND, Gordon, 598748 Singapur (SG); FELDMANN, Joerg, 12683 Berlin (DE); BUCHNER, Dagmar, 12435 Berlin (DE); HINRICHSEN, Lisa, 267951 Singapur (SG)
(74) Representative: Biotronik Corporate Services SE

(57) **Abstract**

An implantable medical device (1) for implantation into a patient comprises a body (100, 150) having a distal end (101, 151) and an electrode device (14) for at least one of emitting an electrical stimulation signal and sensing an electrical sense signal, said electrode device (14) being arranged at said distal end (101, 151) of the body (100, 150) and comprising a helix element (142) to be screwed into tissue and a steroid reservoir (18) provided on the electrode device (14) or the body (100).

## Description

The present invention relates to an implantable medical device for implantation into a patient according to the preamble of claim 1.

An implantable medical device of this kind comprises a body having a distal end, and an electrode device for at least one of emitting an electrical stimulation signal and sensing an electrical sense signal. The electrode device is arranged at the distal end of the body and comprises a helix element to be screwed into tissue.

The implantable medical device may for example be a stimulation device which comprises a generator to be implanted for example subcutaneously at a location remote from the heart. In this case the body is formed for example by a lead extending from the generator into the heart to allow for a stimulation or a sensing of signals at a location of interest within the heart, for example within the right ventricle.

Alternatively, the implantable medical device may be a leadless stimulation device, such as a leadless pacemaker device. In this case the body is formed by the housing of the leadless pacemaker device, which encapsulates components of the leadless pacemaker device such as a processor, a data memory, a battery and other processing equipment to allow for operation of the leadless pacemaker device in an autarkic manner. The leadless pacemaker device may be implanted directly into the heart and may operate within the heart, for example within the right ventricle of the heart, without requiring any leads for placing an electrode at a location of interest within the heart.

With common electrode arrangements of implantable medical devices, an injection of stimulation signals generally is possible at the surface of intra-cardiac tissue, an electrode being in contact with intra-cardiac tissue in order to allow an injection of stimulation energy into the tissue. With new approaches for example for providing e.g. a stimulation in case of a so-called left bundle block, it may be desired to provide for an excitation in a localized fashion in the region of the so-called left bundle branch, which requires to engage with intra-cardiac tissue at the septum of the heart and to place an electrode in the vicinity of the left bundle branch, such that stimulation energy may be specifically injected into the left bundle branch. As this requires a penetration into the septum, there is a general desire to provide for an anchoring of an implantable medical device on intra-cardiac tissue which is easy to establish and allows for an excitation comparatively deep within the tissue, in particular in the context of a left bundle branch pacing.

In particular, when introducing an electrode device for example arranged on a lead into the septum from the right ventricle in order to reach towards the left bundle branch, the electrode device must be inserted into the tissue to reach a substantial depth in order to come to lie in the vicinity of the conductive structures of the left bundle branch. One approach for achieving this may be to use an electrode device comprising a helix element which, for implantation within the heart, is screwed into the septum in order to reach the left bundle branch. As the helix element however may be limited in its axial length in order to ease implantation for example by using a catheter and in order to improve the stability of the electrode device, it may be desirable to have the body of the implantable medical device also penetrate into tissue, such that the helix element may be inserted into the tissue deep enough for electrically contacting conductive structures of the conductive system of the heart.

It is an object of the instant invention to provide an implantable medical device which allows for an easy implantation in order to provide for e.g. a left bundle branch pacing operation.

This object is achieved for means of an implantable medical device comprising the features of claim 1.

In one aspect, an implantable medical device for implantation into a patient comprises: a body having a distal end; an electrode device for at least one of emitting an electrical stimulation signal and sensing an electrical sense signal, such electrode device being arranged at the distal end of the body and comprising a helix element to be screwed into tissue; and a steroid reservoir provided on the electrode device or the body.

In one embodiment, the implantable medical device further comprises a blade section configured to cut into tissue when said helix element is screwed into tissue.

It has been found in trials that in particular the endocardium hinders the body of the implantable medical device to engage with and penetrate into tissue when the body with its distal end is implanted e.g. on the septum of the heart for achieving a left bundle branch stimulation or a stimulation of other conductive structures which are located comparatively deep within the tissue. As the endocardium cannot be easily penetrated by the body, for example being formed by a lead of a sub-cutaneous implanted pacemaker device or by a housing of a leadless pacemaker device, it is required that additional measures are provided in order to allow for a reliable and reproducible engagement of the body with the tissue while screwing the helix element of the electrode device into tissue.

For this purpose, a blade section may be provided, the blade section being configured to cut into tissue when the helix element is screwed into tissue. When the helix element is brought into engagement with the tissue by rotating the body with the helix element arranged thereon, the blade section is moved relative to the tissue and is brought into operative engagement with the tissue, such that the blade section cuts through the tissue and forms an opening within the tissue, for example within the endocardium of the septum of the heart, allowing the body with its distal end to follow the helix element and to penetrate into tissue. **In** this way the helix element may be placed in the tissue at a depth which is larger than the axial length of the helix element (corresponding to the axial length by which the helix element protrudes from the distal end of the body of the implantable medical device), in that the helix element may be further screwed into the tissue once the body with its distal end comes into contact with the tissue by allowing the body of the implantable medical device to enter into the tissue and to engage with the tissue.

In an implanted state, hence, a distal portion of the body may rest within the tissue, while the helix element of the electrode device is placed at a depth within the tissue at which conductive structures deep within the tissue may be contacted for providing for a stimulation and/or sensing at the conductive structures, e.g. at the left bundle branch within the septum of the heart.

In one embodiment, the helix element comprises a first end and a second end, wherein the helix element is connected to the distal end of the body at the second end. The blade section herein, in one embodiment, is arranged in the vicinity of the second end. By arranging the blade section at a location at which the helix element protrudes from the distal end of the body, that is at the axial location of the distal end, it can be achieved that the blade section starts to cut into tissue once the helix element is screwed into tissue with its entire axial length. Once the body with its distal end comes to abut the tissue, the blade section interacts with the tissue and cuts into the tissue, such that upon a further rotation of the body with the helix element arranged thereon the blade section cuts through the tissue and forms an opening within the tissue allowing the body of the implantable medical device to enter into the tissue.

In particular, the blade section may cut through the endocardium when the body is implanted on the septum of the heart for achieving e.g. a left bundle branch pacing, the blade section forming an opening within the endocardium which allows the body to penetrate through the endocardium and to enter into tissue beyond the endocardium.

In one embodiment, the helix element protrudes from the distal end of the body along a longitudinal axis, said helix element being helically wound about the longitudinal axis. Herein, the blade section may be formed to extend in between a portion of the helix element and the distal end of the body, in particular between a portion of the helix element which is in close proximity to the distal end of the body such that the blade section is formed in the vicinity of the distal end of the body.

In one embodiment, the portion of the helix element and the distal end together define an angular space therebetween, wherein the blade section is arranged in the angular space. The portion of the helix element may extend from the distal end of the body approximately at an angle corresponding to the inclination angle of the helical screw formed by the helix element. The portion of the helix element hence forms an angle with the distal end, the angle defining an angular space which axially is delimited by the distal end on the one side and by the portion of the helix element on the other side. In this angular space the blade section is formed, the blade section hence extending in between the distal end and the portion of the helix element and hence within a transitional region in between the distal end and the helix element.

By forming the blade section within the angular space, it in particular may be avoided that tissue may be clamped in between the helix element and the distal end when screwing the helix element into tissue. As the helix element is connected to the distal end of the body and hence extends with respect to the body at an inclined angle according to its helical shape, tissue may be caught in between the helix element and the distal end in particular in that region in which the helix element is connected to the distal end, which however may be avoided by placing the blade section in that particular region and by allowing the blade section to cut through tissue, hence preventing tissue to get caught in between the helix element and the body of the implantable medical device.

The blade section may be formed in many different ways.

The blade section may for example be integrally formed with the body of the implantable medical device. If the body of the implantable medical device for example is formed by injection molding, the blade section may be formed together with the body using an injection molding technique. The body for example may be formed as a whole or in part using a material such as PEEK, TPU, metal, coated metal, silicone, polyamide, polycarbonate, polyimide, LCP, an oxide material such as titanium dioxide or aluminum dioxide, a ceramic material, SiC, or a glass material. The blade section herein may be formed on the body e.g. by a protruding element distally extending from the distal end of the body.

In another embodiment, the blade section may be integrally formed with the helix element. The helix element may for example be formed from a nitinol material, for example from a nitinol wire. The blade section herein may be formed on the helix element e.g. by forming a sharpened edge on the wire of the helix element, for example by grinding a portion of the helix element.

In yet another embodiment, the blade section may be formed by a separate element which is connected to the helix element and/or the body of the implantable medical device, for example using a bonding connection such as a welding or gluing connection.

In one embodiment, the blade section comprises a blade edge which is sharpened to cut into tissue. The blade edge may for example extend substantially longitudinally along a longitudinal direction along which the helix element protrudes from the distal end of the body. The blade edge hence may extend substantially transverse with respect to a rotational direction (pointing about the longitudinal direction) along which the helix element is to be screwed into tissue in order to engage the electrode device with the tissue. By having the blade edge extend transversely with respect to the rotational direction, it is made sure that the blade edge, when rotating the helix element for engaging the helix element with tissue, cuts into and through tissue, hence forming an opening within the tissue into which the body of the implantable medical device may engage for allowing the helix element to be screwed deeper into the tissue.

In one embodiment, the electrode device comprises a pin element configured to engage with tissue, the helix element being connected to the pin element. The pin element may have a pointed tip facing in the distal direction and hence allowing the pin element to pierce into tissue. The pin element may for example be formed from an electrically conductive inner core of the body of the implantable medical device, the inner core forming an inner conductor of the body (which in this case for example may be a lead connected to a generator of the implantable medical device). The helix element, beneficially, protrudes from the pin element in the distal direction, such that the helix element may be screwed into tissue and may be followed by the pin element by having the pin element penetrate into the tissue when screwing the helix element into the tissue.

In one embodiment, a portion of the helix element is wound about the pin element and is connected to the pin element along a connection seam. The connection seam may for example be formed by a bonded connection, for example by a glue seam or a weld seam. Alternatively, the helix element may be integrally formed with the pin element, for example by stamping, rolling or milling. The blade section herein, in one embodiment, is formed on the connection seam, in particular in the vicinity of the axial location of the tip of the pin element.

If the helix element is (with a portion) wound about the pin element and is connected to the pin element along a connection seam, the connection in between the pin element and the helix element is established along a connection line which helically extends about the pin element in between the pin element and the helix element. Approximately at the axial end of the pin element the connection line comes to an end, and the helix element extends and protrudes from the pin element distally along the axial, longitudinal direction. At the end of the connection line the connection seam may form the blade section, for example in that the connection seam forms a sharpened edge at the axial end location of the connection seam. For manufacturing the blade section, the connection seam may for example be sharpened at its axial end, such that the blade section is formed at a bifurcation point at which the helix element departs and extends from the pin element distally along the longitudinal direction. By forming the blade section at this axial location, the blade section may cut into tissue once the helix element has been screwed into tissue as far as the pin element coming into contact with the tissue, such that an opening is formed within the tissue allowing to penetrate the tissue by means of the pin element.

In one embodiment, the body may be formed by a lead which is connectable to a generator of the implantable medical device. In this case, the generator may be implanted into a patient for example subcutaneously remote from the heart, the lead forming the body extending from the generator into the heart such that the body with the electrode device arranged thereon is placed in the heart, for example within the right ventricle in order to engage with tissue at the right ventricle. The distal end is to be implanted into the heart to engage with intra-cardiac tissue for anchoring the body with its distal end on tissue within the heart. By engaging with tissue, herein, the electrode device couples with tissue and hence may be used for at least one of emitting an electrical stimulation signal and sensing an electrical sense signal.

The lead may for example comprise a connector which allows an electrical connection of the lead to the generator. The connector may for example be plugged into a corresponding plug of the generator, wherein the connector may for example comprise an arrangement of contact elements to electrically contact to the generator. The connector may have a standardized shape and may for example be formed as a DF2 or DF4 connector.

In another embodiment, the body may be formed by a housing of a leadless pacemaker device. In this case, the implantable medical device is formed as a leadless device, which does not comprise leads extending from a location outside of the heart into the heart for providing for a stimulation and/or sensing within the heart. The housing of the leadless pacemaker device may be placed on tissue with a distal end formed by the housing, the electrode device being placed on the distal end and engaging with tissue when placing the leadless pacemaker device on tissue with its distal end.

The idea of the invention shall subsequently be described in more detail with reference to the embodiments shown in the figures. Herein:
- Fig. 1: shows a schematic view of the human heart, including the sinoatrial node, the atrioventricular node, the HIS bundle and the left bundle branch and right bundle branch extending from the HIS bundle;
- Fig. 2: shows a schematic drawing of the heart with a lead implanted therein;
- Fig. 3: shows a schematic drawing of the heart with a leadless stimulation device implanted therein;
- Fig. 4: shows a view of an embodiment of an implantable medical device having a body and an electrode device arranged thereon.
- Fig. 5: shows a schematic drawing of an embodiment of an implantable medical device, in the vicinity of a distal end of a body of the implantable medical device, a helix element of an electrode device axially protruding from the distal end, a steroid reservoir and a blade section being formed in between a portion of the helix element and the distal end of the body;
- Fig. 6A: shows a view of an embodiment of an implantable medical device having a body, an electrode device comprising a helix element and a blade section;
- Fig. 6B: shows another view of the embodiment of Fig. 6A;
- Fig. 7A: shows a view of a pre-assembly group of the embodiment of Figs. 6A, 6B;
- Fig. 7B: shows another view of the pre-assembly group of Fig. 7A;
- Fig. 8: shows a schematic view of a body of an implantable medical device having a blade section;
- Fig. 9: shows a view of another embodiment of an implantable medical device;
- Fig. 10: shows a view of an embodiment of an implantable medical device, in the vicinity of a distal end of a body of the implantable medical device, an electrode device comprising a pin element and a helix element;
- Fig. 11: shows a schematic drawing of a blade section formed in between the helix element and the pin element;
- Fig. 12: shows a schematic drawing of another embodiment of an implantable medical device having a blade section formed on a helix element;
- Fig. 13: shows a schematic view of yet another embodiment of an implantable medical device having a blade section; and
- Fig. 14: shows a schematic view of yet another embodiment of an implantable medical device having a blade section.

Subsequently, embodiments of the invention shall be described in detail with reference to the drawings. In the drawings, like reference numerals designate like structural elements.

It is to be noted that the embodiments are not limiting for the invention, but merely represent illustrative examples.

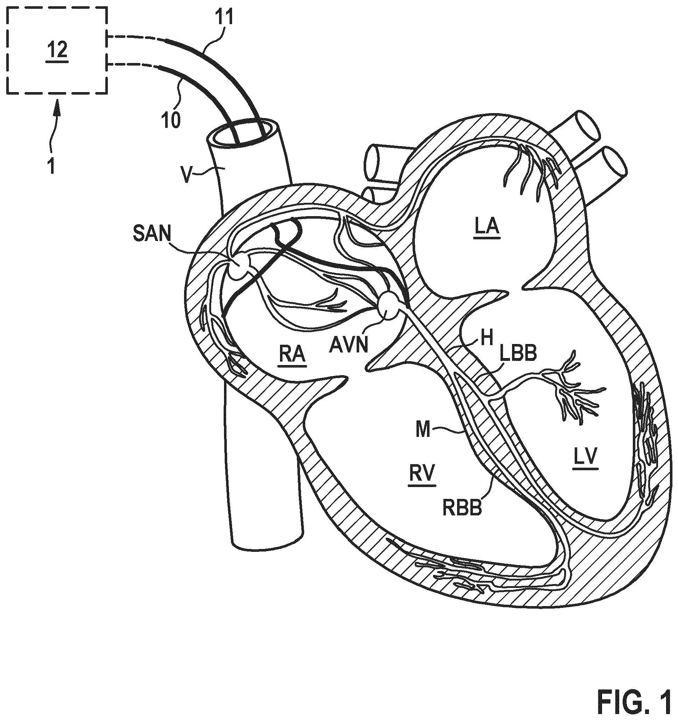

Fig. 1 shows, in a schematic drawing, the human heart comprising the right atrium RA, the right ventricle RV, the left atrium LA and the left ventricle LV, the so-called sinoatrial node SAN being located in the wall of the right atrium RA and being formed by a group of cells having the ability to spontaneously produce an electrical impulse that travels through the heart's electrical conduction system, thus causing the heart to contract in order to pump blood through the heart. The atrioventricular node AVN serves to coordinate electrical conduction in between the atria and the ventricles and is located at the lower back section of the intra-atrial septum near the opening of the coronary sinus. The so-called HIS bundle H extends from the atrioventricular node AVN, the HIS bundle H being comprised of heart muscle cells specialized for electrical conduction and forming part of the electrical conduction system for transmitting electrical impulses from the atrioventricular node AVN via the so-called right bundle branch RBB around the right ventricle RV and via the left bundle branch LBB around the left ventricle LV.

In the embodiment of Fig. 1, an implantable medical device 1 in the shape of a stimulation device, such as a CRT device, is implanted in a patient, the implantable medical device 1 comprising a generator 12 connected to leads 10, 11 extending from the generator 12 through the superior vena V into the patient's heart. By means of the leads 10, 11, electrical signals for providing a pacing action in the heart shall be injected into intra-cardiac tissue potentially at different locations within the heart, and sense signals may be received. In addition, possibly a defibrillation therapy may be performed by an electrode arrangement arranged on one or both of the leads 10, 11.

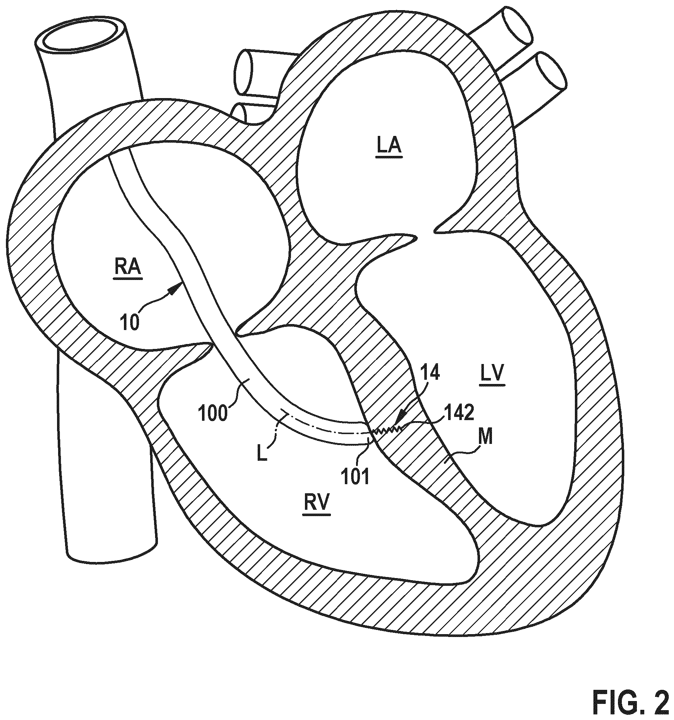

In an embodiment shown in Fig. 2, a lead 10 is implanted into the heart such that it extends into the right ventricle RV of the heart and, at a distal end 101 of a lead body 100, is arranged on intra-cardiac tissue at the septum M in between the right ventricle RV and the left ventricle LV of the heart. At the distal end 101 herein an electrode device 14 comprising a helix element 142 is arranged, the electrode device 14 serving to establish an electrical coupling to tissue and to anchor the lead 10 with its body 100 to tissue in the region of the septum M within the heart.

An implantable medical device 1 as concerned herein may generally be a cardiac stimulation device such as a cardiac pacemaker device. A stimulation device of this kind may comprise a generator 12, as shown in Fig. 1, which may be subcutaneously implanted into a patient at a location remote from the heart, one or multiple leads 10, 11 extending from the generator 12 into the heart for emitting stimulation signals in the heart or for obtaining sense signals at one or multiple locations from the heart.

If the implantable medical device 1 is a stimulation device using leads, a lead 10 forms a generally longitudinal, tubular body 100 extending along a longitudinal axis L, as shown in Fig. 2, which reaches into the heart and is anchored at a location of interest, for example on the septum M of the heart in the region of the right ventricle RV.

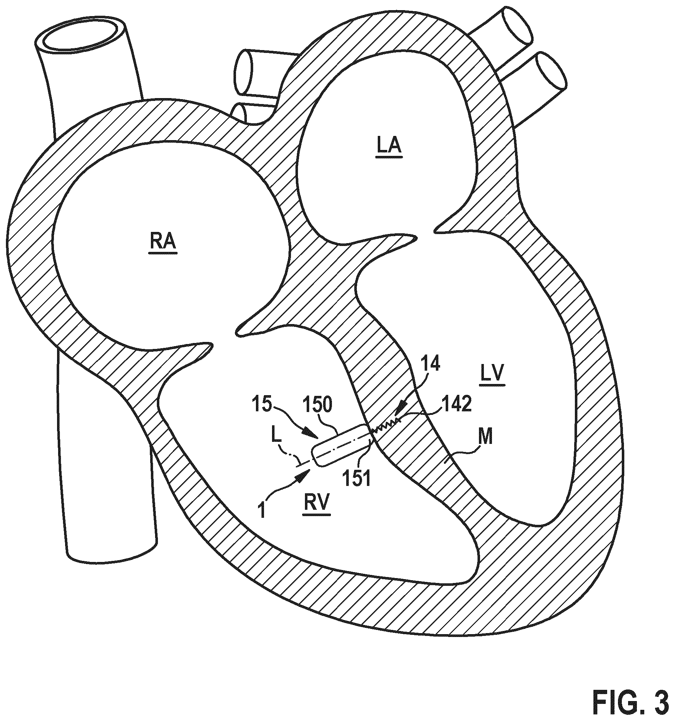

Referring now to Fig. 3, in another embodiment the implantable medical device 1 may be a leadless pacemaker device 15, which does not comprise leads, but has the shape of a capsule and may be directly implanted into the heart, for example into the right ventricle RV of the heart. The leadless pacemaker device 15 comprises a body 150 in the shape of a housing which extends longitudinally along a longitudinal axis L and encapsulates components of the leadless pacemaker device 15, such as a processing device, a data memory, a battery, pulse generation circuitry and the like to allow for a stimulation operation immediately within the heart.

In the embodiment of Fig. 3, the body 150 in the shape of the housing of the leadless pacemaker device 15 forms a distal end 151 which is placed on intra-cardiac tissue in the region of the septum M of the heart. An electrode device 14 comprising a helix element 142 is arranged on the body 150 in the region of the distal end 151 and extends from the distal end 151 generally along the longitudinal axis L.

Although subsequently embodiments are described specifically with reference to implantable medical devices 1 having a lead 10 forming a body 100 which carries an electrode device 14 having a helix element 142, it shall be noted that the subsequent description by no means shall be understood to be limiting, but equally applies also to implantable medical devices 1 formed by leadless pacemaker devices 15.

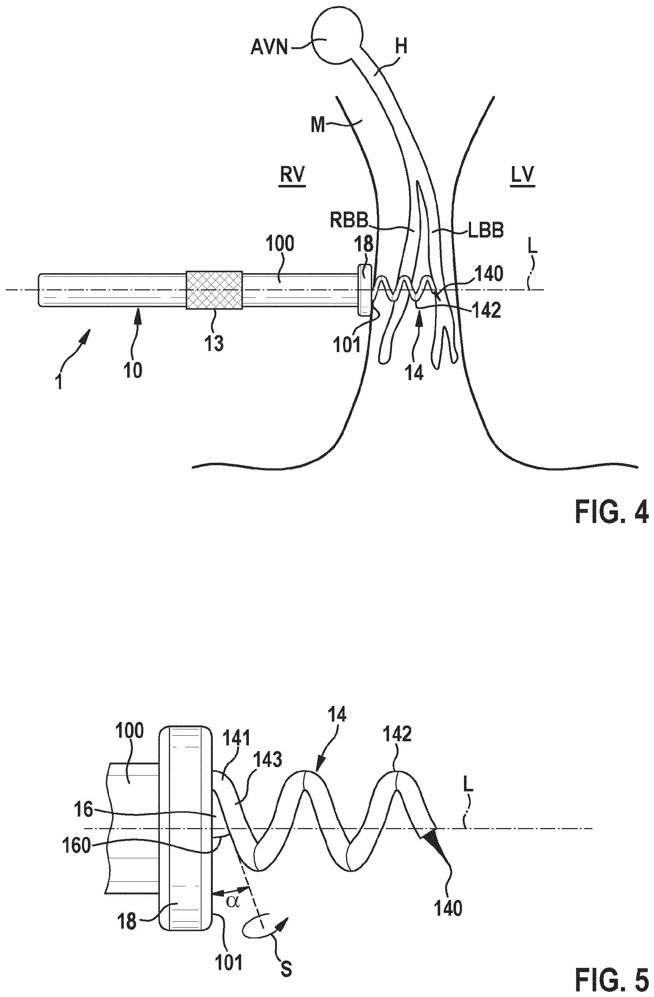

Referring now to Figs. 4 and 5, in one embodiment the electrode device 14 serves to establish an electrical coupling to tissue at the septum M of the heart, the electrode device 14 in particular being shaped such that a coupling to conductive structures deep in the tissue, for example at the left bundle branch LBB, may be established. During operation, herein, an electrical dipole may for example be formed in between the electrode device 14, whose helix element 142 is formed by an electrically conductive wire (e.g. a nitinol wire), and a counter electrode in the shape of a ring electrode 13 placed on the body 100.

When implanting the body 100 of the implantable medical device 1 on tissue, the electrode device 14 with the helix element 142 at the distal end 101 of the body 100 is screwed into tissue, such that the electrode device 14 comes into engagement with the tissue. If a coupling to structures deep in the tissue shall be established, the electrode device 14 herein needs to be screwed into the tissue such that the helix element 142 reaches into the tissue towards and up to the targeted conductive structures, such as the left bundle branch LBB.

A blade section 16 may be used, which is configured to cut into tissue when the electrode device 14 with the helix element 142 is screwed into tissue during the implantation process.

As shown in Fig. 5, the blade section 16 may for example be arranged in between the distal end 101 of the body 100 and a portion 143 of the helix element 142, the helix element 142 being connected to the body 100 at an end 141 and extending axially along a longitudinal axis L from the distal end 101 of the body 100 towards a free end 140.

In particular, the blade section 16 may be formed within an angular space α defined by the distal end 101 of the body 100 and the portion 143 of the helix element 142. The helix element 142 is wound about the longitudinal axis L and comprises an inclination by which the helix element 142 helically is wound about the longitudinal axis L to rise and protrude from the distal end 101 of the body 100 along the longitudinal axis L. By forming the blade section 16 within the angular space α formed in between the distal end 101 and the portion 143 of the helix element 142, the blade section 16 is formed within a transitional region in between the helix element 142 and the body 100, the blade section 16 being arranged in the vicinity of the end 141 of the helix element 142 at which the helix element 142 enters into the body 100 of the implantable medical device 1.

The blade section 16 comprises a blade edge 160 facing in a screwing direction S in which the helix element 142 is to be rotated for screwing the helix element 142 into tissue when implanting the medical device e.g. on the septum M of the heart. Because the blade section 16 is arranged at the axial location of the distal end 101 of the body 100, the blade section 16 comes to interact with tissue once the helix element 142 is fully screwed into the tissue. As the helix element 142 is rotated for screwing it into tissue, the blade section 16 with its blade edge 160 cuts, upon contacting the tissue (e.g. the endocardium at the septum M of the heart), a circular opening into the tissue, the opening having a size substantially equal to or only slightly smaller than the cross-sectional dimensions of the body 100 of the implantable medical device 1. Hence, by cutting an opening into the tissue using the blade section 16, the body 100 is allowed to penetrate into the tissue when further screwing the helix element 142 into the tissue, such that the helix element 142 may further be advanced into the tissue and may be followed by the body 100.

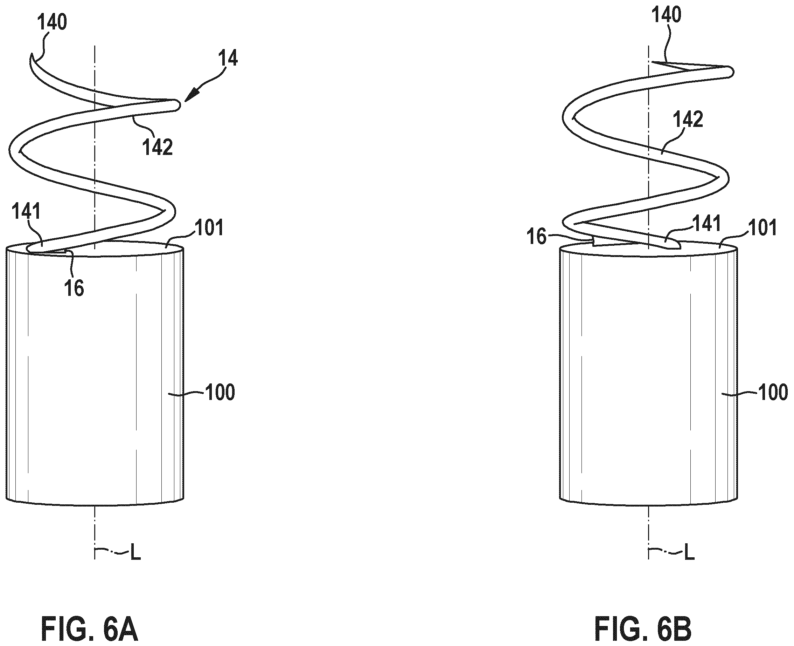

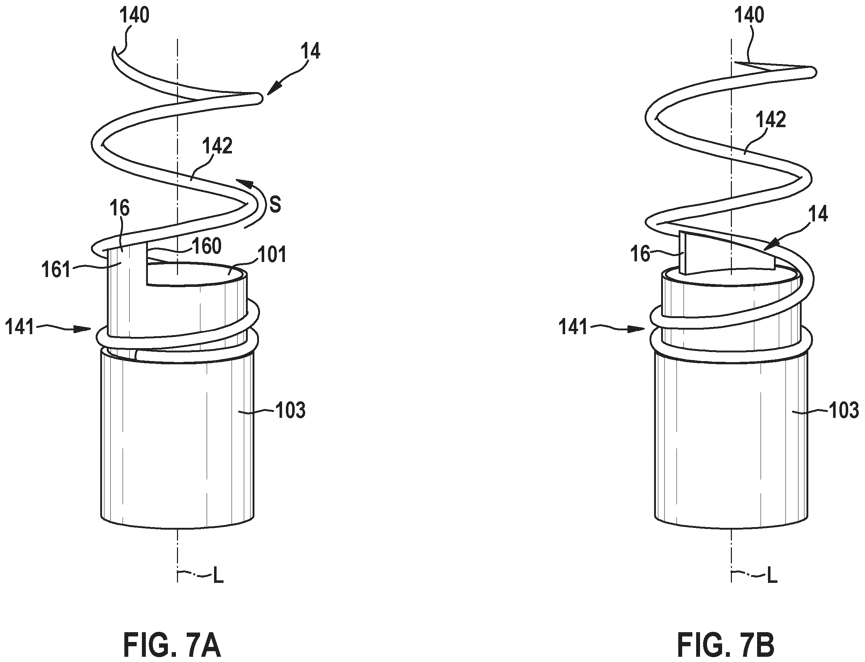

Referring now to Figs. 6A, 6B and 7A, 7B, in one embodiment the helix element 142 is arranged on the body 100 such that the end 141 of the helix element 142 is placed at a radially outer location at the distal end 101 of the body 100. Herein, for manufacturing the arrangement of Figs. 6A, 6B, the helix element 142 may be connected to a shaft element 103, as shown in Figs. 7A, 7B, for example by establishing a welding connection in between the helix element 142 and the shaft 103. The shaft 103 may then be enclosed within a sleeve element to form the body 100, the sleeve element encapsulating the shaft 103 with the end 141 of the helix element 142 arranged thereon. The sleeve element may for example be formed by molding material onto the shaft 103, or by providing a sleeve element as a separate element in which the shaft 103 with the helix element 142 arranged thereon is received.

As visible from Figs. 7A, 7B, the blade section 16, in the shown embodiment, is formed on the shaft 103 and axially protrudes from the distal end 101 formed by the shaft 103. The blade section 16 herein forms a blade edge 160 which substantially extends in parallel to the longitudinal axis L and points into a screwing direction S about which the helix element 142 is to be screwed into tissue for implanting the implantable medical device 1 on the tissue.

The blade section 16 herein forms a guide face on its outside, the guide face facing radially outwards and serving to guide the helix element 142 with respect to the shaft 103. The blade section 16 extends in between the distal end 101 and the most approximate turn of the helix element 142, such that the blade section 16 is arranged within an angular space α in between the most approximate turn of the helix element 142 and the distal end 101 of the body 100.

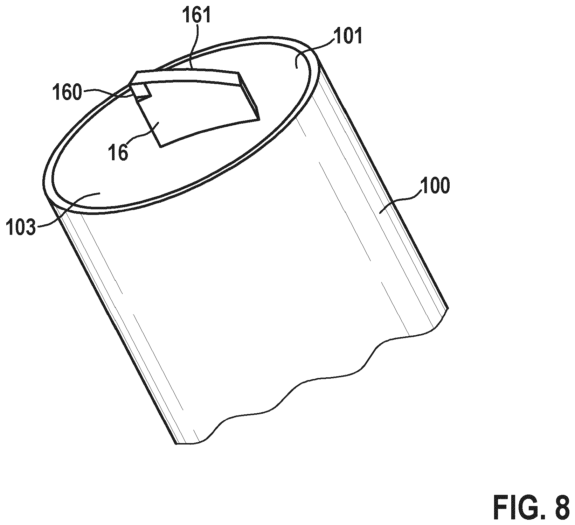

Referring now to Fig. 8, a blade section 16 may be integrally molded with the body 100 to protrude from a distal end 101 of the body 100. In the embodiment of Fig. 8, a helix element 142 of an electrode device 14 may for example be connected to the body 100, wherein a guide face 161 on top of the blade section 16 or on an outer face of the blade section 16 may be configured to guide the helix element 142 with respect to the body 100.

Materials which are used for forming the body 100 may for example include PEEK, TPU, metal, coated metal, silicone, polyamide, polycarbonate, polyimide, LCP, an oxide material such as titanium dioxide or aluminum dioxide, a ceramic material, SiC, or a glass material.

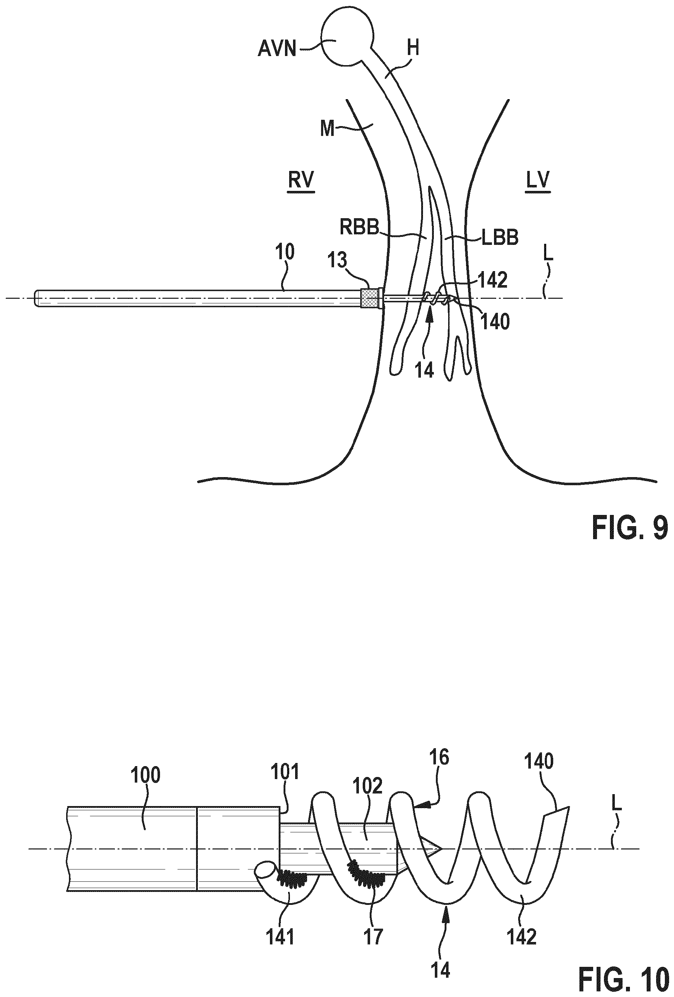

Referring now to Figs. 9 and 10, in another embodiment the electrode device 14 may comprise a pin element 102, which may be formed by an inner, electrical conductor of the body 100. The pin element 102 forms a pointed tip which distally points along the longitudinal axis L, a helix element 142 being partially wound about the pin element 102 and being fixed to the pin element 102 along a connection seam, for example formed by a gluing seam or a weld seam.

In the embodiment of Figs. 9 and 10, when screwing the electrode device 14 with the helix element 142 into tissue, the pin element 102 shall be allowed to engage with tissue in order to insert the electrode device 14 deep into tissue in order to couple to conductive structures deep within the tissue, for example the left bundle branch LBB at the septum M of the heart. In order to ease penetration into the tissue, in particular through the endocardium at the septum M of the heart, a blade section 16 is formed in the vicinity of the axial position of the pointed tip of the pin element 102, the blade section 16 in the shown embodiment being formed for example on the connection seam at an axial end of the connection seam, that is at a bifurcation point at which the helix element 142 extends and protrudes from the pin element 102 axially along the longitudinal axis L.

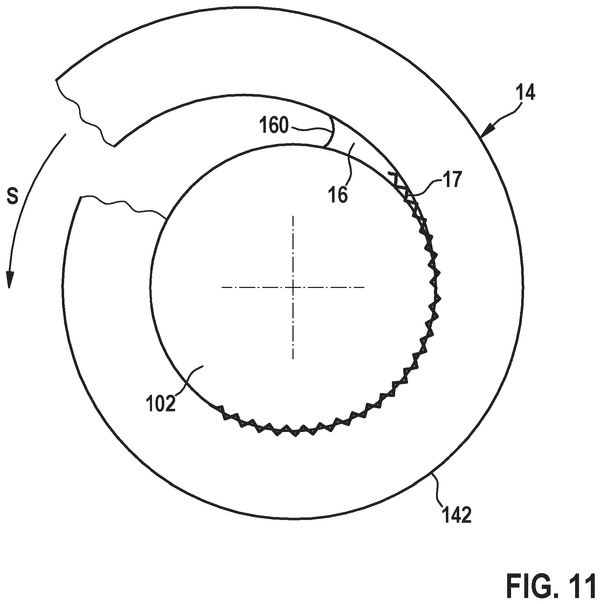

This is illustrated in Fig. 11. Namely, the helix element 142, with its portion wound about the pin element 102, is connected to the pin element 102 along a connection seam 17 which helically extends in between the helix element 142 and the pin element 102 placed radially within the helix element 142. At the axial location of the pointed tip of the pin element 102, the helix element 142 departs from the pin element 102, and the connection seam 17 ends. At this location the connection seam 17 may be sharpened during manufacturing, for example by employing a grinding technique, such that a blade section 16 with a sharpened blade edge 160 is formed, the blade edge 160 facing into the screwing direction S along which the helix element 142 helically extends from the pin element 102.

Hence, at a transitional point between the freely extending portion of the helix element 142 and the pin element 102 a blade section 16 is formed, which cuts into tissue once the helix element 142 is screwed into tissue, during the implantation process, with its portion freely extending from the pin element 102. Once the pin element 102 comes into contact with the tissue, the blade section 16 with its blade edge 160 cuts through the tissue and hence opens the tissue, allowing the pin element 102 entering the tissue and hence allowing the helix element 142 to be screwed into tissue further.

Other embodiments of forming a blade section 16 on an implantable medical device 1 are conceivable.

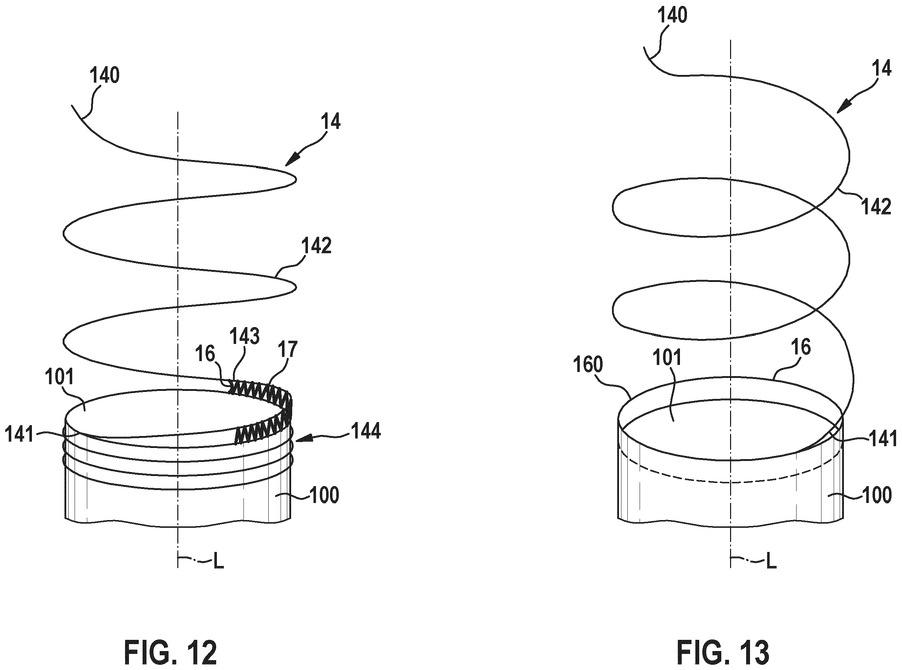

Referring for example to Fig. 12, a helix element 142 may be attached to a body 100 by means of multiple turns 144 formed at the proximal end 141 of the helix element 142. The turns 144 serving to establish the connection with the body 100 herein may be wound about the body 100 such that they are substantially not inclined and rest upon one another to form a connection portion by means of which the helix element 142 is connected to the body 100.

In between a last turn 144 (when viewed along a distal direction towards the free, distal end 140 of the helix element 142) which is substantially not inclined and a portion 143 of a first turn of the helix element 142 being inclined with respect to the distal end 101 of the body 100 and protruding from the distal end 101, for example a weld connection 17 is formed, the weld connection 17 at its front end forming a blade section 16 serving to cut into tissue when screwing the helix element 142 into tissue during the implantation process.

In yet another embodiment, shown in Fig. 13, a blade section 16 having a circular blade edge 160 may be formed at the distal end 101 of the body 100, the blade edge 160 serving to cut into tissue substantially in a stamping action when bringing the distal end 101 into abutment with tissue. The helix element 142 axially protrudes from the distal end 101, such that the helix element 142 may be screwed into tissue for implanting the implantable medical device 1 e.g. on the septum M of the heart.

In yet another embodiment, the blade section 16 may immediately be formed e.g. on the helix element 142, for example by sharpening a portion of the wire of the helix element 142.

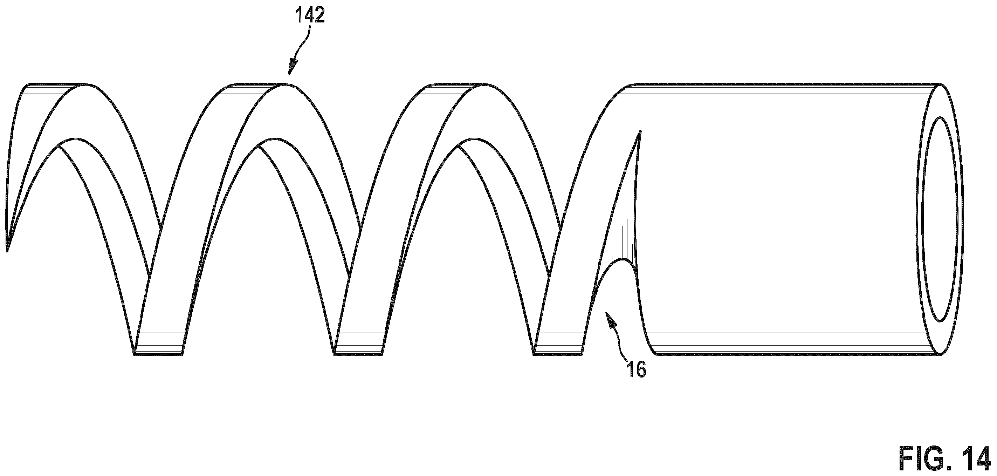

In yet another embodiment, shown in Fig. 14, a helix element 142 may be cut into a pipe using a laser. During this laser cutting process, a blade section 16 is also formed out of the pipe together with the helix element 142. A blade edge 160 of the blade section 16 can be either formed by laser cutting or by grinding. The remaining part of the pipe may form the distal end 101 of the body 100.

The helix element 142, in one embodiment, may for example protrude from the distal end 101 of the body 100 axially along a length in between 1 mm and 10 mm, for example in between 2 mm and 5 mm.

A blade edge 160 of the blade section 16 may for example have a length in between 0.05 and 1 mm, for example when arranging the blade section 16 in an angular space α in between the distal end 101 and an adjoining portion 143 of the helix element 142, as shown in Fig. 5.

In one embodiment, a ring electrode may be provided on the body 100, the ring electrode for example being longitudinally displaceable on the body 100. The ring electrodes herein may serve to limit the penetration depth of the body 100.

In another embodiment, a ring element or a stent element may be pushed over the body 100 once the helix element 142 is screwed into tissue to reach a sufficient depth. The ring element or stent element may serve as a limiting feature in order to prevent a further penetration of tissue by the body 100. A ring element could for example be made from a silicone material and may be non-displaceably held on the body 100 due to friction in between the ring element and the body 100.

In yet another embodiment, a ring element may be pre-mounted on the body 100. The ring element may be designed such that it can only move proximally on the body 100 and positions itself on the body 100 as the body 100 enters into tissue. Once implantation is complete, the ring element may be fixed to the body 100, hence preventing a further penetration of the tissue.

In yet another embodiment, a silicone tubing or an injection molded part may be glued or otherwise fixed to the distal end 101 of the body 100, the silicone tubing or injection molded part e.g. distally protruding from the body 100 and functioning as a pinch bushing. Such element may serve to control the screwing process, in that a resistance for penetration is increased the farther the helix element 142 is screwed into tissue, allowing for a fine adjustment of a screwing depth, and reducing a risk for a perforation.

As shown in Figs. 4 and 5, a steroid reservoir 18 is provided on the body 100. Alternatively, the steroid reservoir may be provided on the electrode device 14. The steroid reservoir is formed as a collar on the body 100. Alternatively, the steroid reservoir may for example be formed as a plug inside the body 100, as a collar on the helix element 142, or as a coating made of a steroid-bearing matrix material provided on the body 100 of the helix element 142.

The idea of the invention is not limited to the embodiments described above.

The implantable medical device may have the shape of a stimulation device comprising leads, or may have the shape of a leadless stimulation device.

### List of Reference Numerals

- 1: Implantable medical device
- 10: Lead
- 100: Lead body
- 101: Distal end
- 102: Inner core (conductor)
- 103: Shaft
- 11: Lead
- 12: Generator
- 13: Electrode
- 14: Electrode device
- 140, 141: End
- 142: Helix element
- 143: Portion
- 144: Turns
- 15: Leadless device
- 150: Body (housing)
- 151: Distal end
- 16: Blade section
- 160: Blade edge
- 161: Guide face
- 17: Weld connection
- 18: Steroid reservoir
- α: Angular space
- AVN: Atrioventricular node
- H: HIS bundle
- L: Longitudinal axis
- LA: Left atrium
- LBB: Left bundle branch
- LV: Left ventricle
- M: Intra-cardiac tissue (myocardium)
- RA: Right atrium
- RBB: Right bundle branch
- RV: Right ventricle
- S: Screwing direction
- SAN: Sinoatrial node
- V: Superior vena

## Claims

1. An implantable medical device (1) for implantation into a patient, comprising:
- a body (100, 150) having a distal end (101, 151);
- an electrode device (14) for at least one of emitting an electrical stimulation signal and sensing an electrical sense signal, said electrode device (14) being arranged at said distal end (101, 151) of the body (100, 150) and comprising a helix element (142) to be screwed into tissue; and
- a steroid reservoir (18) provided on the electrode device (14) or the body (100).

2. The implantable medical device (1) according to claim 1, wherein said helix element (142) comprises a first end (140) and a second end (141), wherein said helix element (142) is connected to the body (100, 150) at the second end (141).

3. The implantable medical device (1) according to one of the preceding claims, wherein said helix element (142) protrudes from the distal end (101, 151) of the body (100, 150) along a longitudinal axis (L), said helix element (142) being helically wound about the longitudinal axis (L).

4. The implantable medical device (1) according to one of the preceding claims, wherein the electrode device (14) comprises a pin element (102) configured to engage with tissue, the helix element (142) being connected to the pin element (102).

5. The implantable medical device (1) according to claim 4, wherein at least a portion of the helix element (142) is wound about the pin element (102) and is connected to the pin element (102) along a connection seam (17).

6. The implantable medical device (1) according to one of the preceding claims, wherein the body (100) is formed by a lead (10) which is connectable to a generator (12) of the implantable medical device (1).

7. The implantable medical device (1) according to one of the preceding claims, wherein the body (150) is formed by a housing of a leadless pacemaker device (15).

8. The implantable medical device (1) according to one of the preceding claims, wherein the steroid reservoir is formed as a plug inside the body (100), as a collar (18) on the body (100) or the helix element (142), or as a coating made of a steroid-bearing matrix material provided on the body (100) of the helix element (142)

9. The implantable medical device (1) according to one of the preceding claims, further comprising a blade section (16) configured to cut into tissue when said helix element (142) is screwed into tissue.

10. The implantable medical device (1) according to claim 9, wherein said helix element (142) protrudes from the distal end (101, 151) of the body (100, 150) along a longitudinal axis (L), said helix element (142) being helically wound about the longitudinal axis (L); and wherein the blade section (16) is formed to extend in between a portion (143) of the helix element (142) and the distal end (101, 151) of the body (100, 150).

11. The implantable medical device (1) according to claim 9 or 10, wherein said blade section (16) is arranged in the vicinity of the second end (141).

12. The implantable medical device (1) according to one of claims 9 to 11, wherein said portion (143) of the helix element (142) and the distal end (101, 151) define an angular space (α) therebetween, the blade section (16) being arranged in said angular space (α).

13. The implantable medical device (1) according to one of claims 9 to 12, wherein the blade section (16) is integrally formed with the body (100, 150) or the helix element (142).

14. The implantable medical device (1) according to one of claims 9 to 13, wherein the blade section (16) comprises a blade edge (160) which is sharpened to cut into tissue.

15. The implantable medical device (1) according to claim 14, wherein the blade edge (160) extends longitudinally along a longitudinal direction along which the helix element (142) protrudes from the distal end (101, 151) of the body (100, 150).
